(19) Europäisches Patentamt
European Patent Office
Office européen des brevets

(11) Veröffentlichungsnummer : **0 057 366**
**B1**

(12) # EUROPÄISCHE PATENTSCHRIFT

(45) Veröffentlichungstag der Patentschrift : **03.04.85**

(51) Int. Cl.⁴ : **C 07 D213/14**, C 07 D213/08

(21) Anmeldenummer : **82100309.2**

(22) Anmeldetag : **18.01.82**

(54) **Verfahren zur Herstellung von Pyridinen.**

(30) Priorität : **30.01.81 DE 3103108**

(43) Veröffentlichungstag der Anmeldung : **11.08.82 Patentblatt 82/32**

(45) Bekanntmachung des Hinweises auf die Patenterteilung : **03.04.85 Patentblatt 85/14**

(84) Benannte Vertragsstaaten : **BE CH DE FR GB LI**

(56) Entgegenhaltungen :
**DE-A- 1 570 006**
**DE-C- 944 250**
**GB-A- 1 110 865**
**GB-A- 1 233 643**

(73) Patentinhaber : **BASF Aktiengesellschaft**
**Carl-Bosch-Strasse 38**
**D-6700 Ludwigshafen (DE)**

(72) Erfinder : **Merger, Franz, Dr.**
**Max-Slevogt-Strasse 25**
**D-6710 Frankenthal (DE)**
Erfinder : **Fouquet, Gerd, Dr.**
**Maxburgstrasse 2**
**D-6730 Neustadt (DE)**

**EP 0 057 366 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von Pyridinen durch Umsetzung von 2-Alkoxy-2,3-dihydro-4H-pyranen oder Glutaraldehyden mit Ammoniumnitrat in Gegenwart von aliphatischen Carbonsäuren.

Die britische Patentschrift 1 102 261 beschreibt ein Verfahren zur Herstellung von Pyridinen durch Umsetzung von Glutaraldehyd, substituiertem Glutaraldehyd oder 2-Alkoxy-2,3-dihydro-4H-pyranen mit Ammoniak oder Ammoniumsalzen in Gegenwart von Sauerstoff und Kupfer (II)- bzw. Eisen (III)-halogeniden. Bezogen auf den eingesezten Dialdehyd werden Pyridin-Selektivitäten von 44 % erzielt. Neben der mäßigen Ausbeute hat das Verfahren den Nachteil, daß bei der Reaktion bezüglich Aldehyd 44 Molprozent Kupfer (II)-chlorid und 542 Molprozent Ammoniumchlorid eingesetzt werden, die zu Nebenprodukten (Chlorpyridinen) führen, wodurch die Reindarstellung des gewünschten, nicht halogenierten Pyridins erschwert wird.

Die GB-A-1 110 865 beschreibt eine Herstellung von Pyridinen durch Umsetzung von unsubstituiertem, substituiertem Glutaraldehyd oder 2-Alkoxy-3,4-dihydro-2-H-pyranen in flüssiger Phase mit Ammoniak oder Ammoniumionen in Gegenwart von molekularem Sauerstoff und Kupferionen bei 80 bis 170 °C. Während der Reaktion wird ein Gemisch von Sauerstoff und Stickstoff durch das Reaktionsgemisch geleitet. Der Sauerstoff muß in solchen Mengen zugeführt werden, daß ein bestimmter Sauerstoffpartialdruck nicht unterschritten wird. In den Beispielen wird als Ammoniumsalz Ammoniumacetat verwendet. Als Reaktionsmedium können Gemische von Wasser und Alkanoylsäuren (in den Beispielen Wasser/Essigsäure) eingesetzt werden.

GB-A-1 233 643 beschreibt eine sehr ähnliche Arbeitsweise der Pyridinherstellung, bei der 3,4-Dihydro-2H-pyran mit Ammoniak oder Ammoniumionen in flüssiger Phase und in Anwesenheit von molekularem Sauerstoff bei einem bestimmten Partialdruck $O_2$ bei 15 bis 150 °C umgesetzt wird. Auch bei diesem Verfahren werden, wie alle Beispiele zeigen, Kupfersalze, Ammoniumacetat und wäßrige Essigsäure verwendet und muß Sauerstoff laufend durch das Reaktionsmedium geführt werden.

Eine entsprechende Synthese zeigt DE-A-1 570 006, die in ihren Beispielen ebenfalls Ammoniumacetat, wäßrige Essigsäure, Kupfersalze, die vorgenannten 2-Alkoxy-3,4-dihydro-2H-pyrane als Ausgangsstoffe, Zirkulation von molekularem Sauerstoff durch das Reaktionsgemisch und einen Temperaturbereich von 15 bis 150 °C verwendet. Statt Kupfersalz wird auch Eisenacetat, statt Essigsäure auch Dimethylacetamid, statt Ammoniumacetat auch Harnstoff oder Formamid eingesetzt. Beispiel 9 zeigt, daß eine Umsetzung ohne Zusatz von Metallsalzen nur eine Ausbeute von 15 % liefert. Auch Edelmetallkatalysatoren werden verwendet.

Es wurde nun gefunden, daß man Pyridine der Formel I

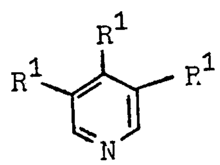

(I)

worin die einzelnen Reste $R^1$ gleich oder verschieden sein können und jeweils ein Wasserstoffatom oder einen aliphatischen Rest bedeuten, durch Umsetzung von

a) 2-Alkoxy-2,3-dihydro-4H-pyranen der Formel II

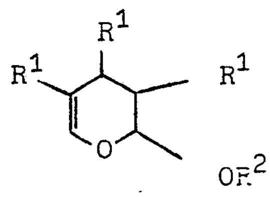

(II) oder

b) Glutaraldehyden der Formel III

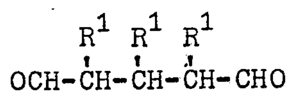

(III)

worin $R^1$ die vorgenannte Bedeutung besitzt und $R^2$ einen aliphatischen Rest bezeichnet, mit Ammoniumsalzen in Gegenwart von aliphatischen Carbonsäuren vorteilhaft erhält, wenn man die Umsetzung mit Ammoniumnitrat in Abwesenheit von Kupfersalzen und in Gegenwart von katalytischen Mengen Salpetersäure durchführt.

Die Umsetzung kann für den Fall der Verwendung von 2,3-Dihydro-2-methoxy-4H-pyran oder Glutaraldehyd durch die folgenden Formeln wiedergegeben werden :

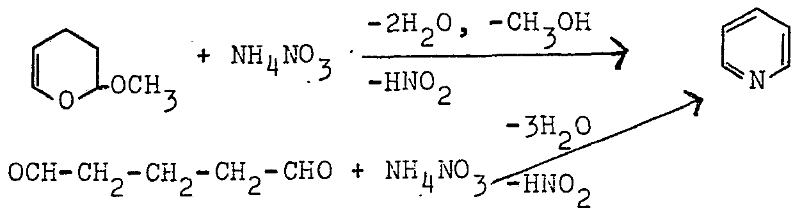


Im Vergleich zu dem bekannten Verfahren liefert das Verfahren nach der Erfindung auf einfacherem und wirtschaftlicherem Wege Pyridine in besserer Raum-Zeit-Ausbeute, Ausbeute und Reinheit. Überraschenderweise kann dabei auf Sauerstoff und/oder andere Oxidationsmittel, wie Salze des dreiwertigen Eisens oder Mangans oder des zweiwertigen Kupfers verzichtet werde, woraus eine wesentlich vereinfachte Aufarbeitung sowie Isolierung des Reaktionsproduktes resultiert. Das Verfahren ist außerdem besonders umweltfreundlich, da zu seiner Durchführung keine abwasserbelastenden Schwermetallsalze benötigt werden.

Die Ausgangsstoffe II und III können mit Ammoniumnitrat in stöchiometrischer Menge oder im Überschuß, zweckmäßig in einem Verhältnis von 1 bis 2, insbesondere 1 bis 1,2 Mol Ammoniumnitrat je Mol Ausgangsstoff II oder III, umgesetzt werden. Bevorzugte Ausgangsstoffe II und III und dementsprechend bevorzugte Endstoffe I sind solche, in deren Formeln die einzelnen Reste $R^1$ gleich oder verschieden sein können und jeweils ein Wasserstoffatom oder einen Alkylrest mit 1 bis 4 Kohlenstoffatomen bedeuten, die einzelnen Reste $R^1$ und $R^2$ gleich oder verschieden sein können, $R^2$ einen Alkylrest mit 1 bis 4 Kohlenstoffatomen bedeutet.

So kommen beispielsweise folgende Ausgangsstoffe II in Betracht : In 3-, 4- oder 5-Stellung einfach oder in 3,4-, 4,5-, 3,5-Stellung gleich oder unterschiedlich zweifach oder in 3-, 4-, 5-Stellung gleich oder unterschiedlich 3-fach durch Methyl-, Ethyl-, Propyl-, Isopropyl-, Butyl-, Isobutyl-, sek.-Butyl-, tert.-Butylgruppen substituierte 2,3-Dihydro-2-methoxy-4H-pyrane : homolog durch vorgenannte Substituenten substituierte oder unsubstituierte 2-Ethoxy-, 2-Propoxy-, 2-Isopropoxy-, 2-Butoxy-, 2-Isobutoxy-, 2-sek.-Butoxy-, 2-tert.-Butoxy-4H-pyrane.

Beispielsweise sind folgende Ausgangsstoffe III geeignet : In 2-, 3-, 4-Stellung einfach oder in 2,3-, 3,4-, 2,4-Stellung gleich oder unterschiedlich zweifach oder in 2,3,4-Stellung gleich oder unterschiedlich 3-fach durch Methyl-, Ethyl-, Propyl-, Isopropyl-, Butyl-, Isobutyl-, sek.-Butyl-, tert.-Butyl-gruppen substituierte Glutaraldehyde ; unsubstituierter Glutaraldehyd.

Die Umsetzung wird in Gegenwart von aliphatischen Carbonsäuren als Lösungsmittel, vorteilhaft mit einer Menge von 10 bis 50, insbesondere von 15 bis 30 Molen Lösungsmittel, bezogen auf 1 Mol Ausgangsstoff II oder III, durchgeführt. Beispielsweise sind folgende Säuren geeignet : Chloressigsäure, Essigsäure, Propionsäure, Buttersäure, Isobuttersäure. Die Säuren können in konzentrierter Form, im Gemisch miteinander und/oder mit einem Lösungsmittel, insbesondere Wasser, angewendet werden. In der Regel enthalten die Säuren mindestens 2 Kohlenstoffatome. Bevorzugt sind Essigsäure, Propionsäure, Buttersäure und Isobuttersäure.

Die Umsetzung wird im allgemeinen bei einer Temperatur von 50 bis 150, bevorzugt 80 bis 130, insbesondere 100 bis 120 °C, drucklos oder unter Druck, kontinuierlich oder diskontinuierlich durchgeführt. In der Regel verwendet man keine organischen Lösungsmittel, aber zweckmäßig Wasser, vorteilhaft 50 bis 200, insbesondere 60 bis 100 Gewichtsprozent Wasser je Gewichtsmenge Ausgangsstoff II oder III.

In einer bevorzugten Ausführungsform verwendet man katalytische Mengen Salpetersäure als Katalysator in Gestalt einer 20- bis 60-, insbesondere 40- bis 50-gewichtsprozentigen, wäßrigen Salpetersäure und in einer Menge von 0,001 bis 0,1, insbesondere 0,01 bis 0,05 Mol Salpetersäure je Mol Ausgangsstoff II oder III.

Die Reaktion kann wie folgt durchgeführt werden : Ein Gemisch von Ausgangsstoff II oder III, aliphatischer Carbonsäure, Wasser, Ammoniumnitrat, Salpetersäure, wird während 15 bis 20 Minuten zu der aliphatischen Carbonsäure bei der Reaktionstemperatur zugegeben. Dann wird der Endstoff in üblicher Weise, z. B. durch Destillation, Ammoniak- oder Natronlauge-zugabe, Destillation oder Extraktion, z. B. mit Ether, und Destillation, isoliert.

Die nach dem Verfahren der Erfindung herstellbaren Pyridine I sind wertvolle Ausgangsstoffe für die Herstellung von Farbstoffen, Pharmazeutika, Schädlingsbekämpfungsmitteln und sind wertvolle Lösungsmittel. Bezüglich der Verwendung wird auf die vorgenannte Literatur und Ullmanns Encyklopädie der technischen Chemie, Band 14, Seite 467, verwiesen.

Die in den folgenden Beispielen genannten Teile bedeuten Gewichtsteile.

## Beispiel 1

142 Teile 5-Ethyl-2,3-dihydro-2-methoxy-4H-pyran werden in 27 Teilen Wasser, 300 Teilen Essigsäure

und 2 Teilen 60-prozentiger Salpetersäure bei 22 °C gelöst. Nach 15 Minuten gibt man eine Lösung von 80 Teilen Ammoniumnitrat in 80 Teilen Wasser zu. Diese Lösung läßt man bei 110 °C zu 800 Teilen Essigsäure unter Rühren innerhalb 20 Minuten zufließen. Man läßt die Reaktionsmischung erkalten, destilliert Essigsäure und Wasser ab, gibt zu dem Rückstand 200 Teile Wasser und macht die Lösung mit einer 25-prozentigen Ammoniaklösung alkalisch. Das 3-Ethylpyridin wird mit Ether extrahiert ; durch Destillation gewinnt man 91 Teile (85 % der Theorie) 3-Ethylpyridin, Sdp. 60 °C/20 mbar.

Beispiel 2

114 Teile 2,3-Dihydro-2-methoxy-4H-pyran werden in 27 Teilen Wasser, 300 Teilen Essigsäure und 2 Teilen 60-prozentiger Salpetersäure bei 22 °C gelöst. Nach 15 Minuten gibt man eine Lösung von 80 Teilen Ammoniumnitrat in 80 Teilen Wasser zu. Diese Lösung gibt man bei 110 °C zu 800 Teilen Essigsäure unter Rühren innerhalb 20 Minuten zu. Von der erkalteten Reaktionsmischung destilliert man Essigsäure und Wasser ab, gibt zu dem Rückstand 200 Teile Wasser und macht die Lösung mit einer 25-prozentigen Ammoniaklösung alkalisch. Pyridin wird mit Ether extrahiert. Durch Destillation gewinnt man 58 Teile (74 % der Theorie) Pyridin, Sdp. 115 °C.

Beispiel 3

Eine Lösung von 128 Teilen 2-Ethylglutaraldehyd (gelöst in 200 Teilen Essigsäure), 88 Teile Essigsäure, 88 Teile Ammoniumnitrat und 60 Teile Wasser läßt man zu 800 Teilen Essigsäure bei 110 bis 115 °C innerhalb 20 Minuten zufließen. Die Reaktionsmischung läßt man erkalten, destilliert Essigsäure und Wasser ab, gibt zu dem Rückstand 100 Teile Wasser und macht die Lösung mit einer 25-prozentigen Ammoniaklösung alkalisch. Das 3-Ethylpyridin wird mit Ether extrahiert ; durch Destillation gewinnt man 93 Teile (87 % der Theorie) 3-Ethylpyridin, Sdp. 60 °C/20 mbar.

Beispiel 4

71 Teile 3-Ethyl-2,3-dihydro-2-methoxy-4H-pyran werden in 13 Teilen Wasser, 150 Teilen Propionsäure und 2 Teilen 60-prozentiger Salpetersäure bei 22 °C gelöst. Nach 15 Minuten läßt man diese Lösung gleichzeitig mit 40 Teilen Ammoniumnitrat in 30 Teilen Wasser zu 400 Teilen Propionsäure bei 120 bis 140 °C innerhalb 20 Minuten zufließen.

Die Reaktionsmischung läßt man erkalten, destilliert Propionsäure, Wasser und Methanol ab, gibt zu dem Rückstand 75 Teile Wasser und macht die Lösung mit einer 10-prozentigen Natronlauge alkalisch. Das 3-Ethylpyridin wird mit Ether extrahiert ; durch Destillation gewinnt man 35 Teile (66 % der Theorie) 3-Ethylpyridin, Sdp. 60 °C/20 mbar.

Beispiel 5

142 Teile 2,3-Dihydro-2-methoxy-3,4-dimethyl-4H-pyran werden in 25 Teilen Wasser, 300 Teilen Essigsäure und 2 Teilen 60-prozentiger Salpetersäure bei 22 °C gelöst. Nach 15 Minuten gibt man dazu eine Lösung von 80 Teilen Ammoniumnitrat in 80 Teilen Wasser. Diese Lösung gibt man bei 110 °C zu 800 Teilen Essigsäure unter Rühren innerhalb 20 Minuten zu. Von der erkalteten Reaktionsmischung destilliert man Essigsäure, Methanol und Wasser ab, gibt zu dem Rückstand 150 Teile Wasser und macht die Lösung mit einer 25-prozentigen Ammoniaklösung alkalisch. 3,4-Dimethylpyridin wird mit Ether extrahiert. Durch Destillation gewinnt man 87 Teile (81 % der Theorie) 3,4-Dimethylpyridin, Sdp. 68 °C/20 mbar.

Beispiel 6

128 Teile 2,3-Dihydro-2-methoxy-3-methyl-4H-pyran werden in 25 Teilen Wasser, 250 Teilen Essigsäure und 2 Teilen 60-prozentiger Salpetersäure bei 22 °C gelöst. Nach 15 Minuten gibt man dazu eine Lösung von 80 Teilen Ammoniumnitrat in 80 Teilen Wasser. Diese Lösung gibt man bei 110 °C zu 800 Teilen Essigsäure unter Rühren innerhalb 20 Minuten zu. Von der erkalteten Reaktionsmischung destilliert man Essigsäure, Wasser und Methanol ab, gibt zu dem Rückstand 200 Teile Wasser und macht die Lösung mit einer 25-prozentigen Ammoniaklösung alkalisch. Das 3-Methylpyridin wird mit Ether extrahiert ; durch Destillation gewinnt man 77 Teile (83 % der Theorie) 3-Methylpyridin, Sdp. 143 °C.

Beispiel 7

142 Teile 2-Ethoxy-2,3-dihydro-4-methyl-4H-pyran werden in 25 Teilen Wasser, 300 Teilen Essigsäure und 2 Teilen 60-prozentiger Salpetersäure bei 22 °C gelöst. Nach 15 Minuten gibt man dazu eine Lösung von 80 Teilen Ammoniumnitrat in 80 Teilen Wasser. Diese Lösung gibt man bei 110 °C zu 800 Teilen Essigsäure unter Rühren innerhalb 20 Minuten zu. Die Reaktionsmischung läßt man erkalten, destilliert Essigsäure, Wasser und Ethanol ab, gibt zu dem Rückstand 200 Teile Wasser und macht die Lösung mit

einer 25-prozentigen Ammoniaklösung alkalisch. 4-Methylpyridin wird mit Ether extrahiert. Durch Destillation gewinnt man 73 Teile (79 % der Theorie) 4-Methylpyridin, Sdp. 143 °C.

Beispiel 8

142 Teile 2,3-Dihydro-2-methoxy-3,5-dimethyl-4H-pyran werden in 25 Teilen Wasser, 300 Teilen Essigsäure und 2 Teilen 60-prozentiger Salpetersäure bei 22 °C gelöst. Nach 15 Minuten gibt man dazu eine Lösung von 80 Teilen Ammoniumnitrat in 80 Teilen Wasser. Diese Lösung läßt man bei 110 °C zu 800 Teilen Essigsäure unter Rühren innerhalb 20 Minuten zufließen. Die Reaktionsmischung läßt man erkalten, destilliert Essigsäure, Wasser und Methanol ab, gibt zu dem Rückstand 200 Teile Wasser und macht die Lösung mit einer 25-prozentigen Ammoniaklösung alkalisch. 3,5-Dimethylpyridin wird mit Ether extrahiert. Durch Destillation gewinnt man 77 Teile (72 % der Theorie) 3,5-Dimethylpyridin, Sdp. 172 °C.

Beispiel 9

156 Teile 3-Ethyl-2,3-dihydro-2-methoxy-5-methyl-4H-pyran werden in 25 Teilen Wasser, 300 Teilen Essigsäure und 2 Teilen 60-prozentiger Salpetersäure bei 22 °C gelöst. Nach 15 Minuten gibt man eine Lösung von 80 Teilen Ammoniumnitrat in 80 Teilen Essigsäure unter Rühren innerhalb 20 Minuten zu. Die Reaktionsmischung läßt man erkalten, destilliert Essigsäure, Wasser und Methanol ab, gibt zu dem Rückstand 200 Teile Wasser und macht die Lösung mit einer 25-prozentigen Ammoniaklösung alkalisch. 3-Ethyl-5-methyl-pyridin wird mit Ether extrahiert. Durch Destillation gewinnt man 80 Teile (66 % der Theorie) 3-Ethyl-5-methyl-pyridin, Sdp. 83 °C/20 mbar.

Beispiel 10

170 Teile 3,5-Diethyl-2,3-dihydro-2-methoxy-4H-pyran werden in 25 Teilen Wasser, 300 Teilen Essigsäure und 2 Teilen 60-prozentiger Salpetersäure bei 22 °C gelöst. Nach 15 Minuten gibt man eine Lösung von 88 Teilen Ammoniumnitrat in 80 Teilen Wasser zu. Diese Lösung läßt man bei 110 °C zu 800 Teilen Essigsäure unter Rühren innerhalb 20 Minuten zufließen. Die Reaktionsmischung läßt man erkalten, destilliert Essigsäure, Wasser und Methanol ab, gibt zu dem Rückstand 200 Teile Wasser und mach die Lösung mit einer 25-prozentigen Ammoniaklösung alkalisch. 3,5-Diethylpyridin wird mit Ether extrahiert. Durch Destillation gewinnt man 87 Teile (64 % der Theorie) 3,5-Diethylpyridin, Sdp. 92 °C/20 mbar.

**Anspruch**

Verfahren zur Herstellung von Pyridinen der Formel I

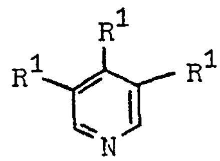

(I)

worin die einzelnen Reste $R^1$ gleich oder verschieden sein können und jeweils ein Wasserstoffatom oder einen aliphatischen Rest bedeuten, durch Umsetzung von

a) 2-Alkoxy-2,3-dihydro-4H-pyranen der Formel II

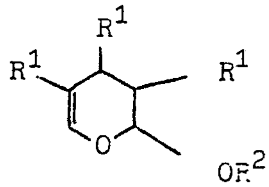

(II) oder

b) Glutaraldehyden der Formel III

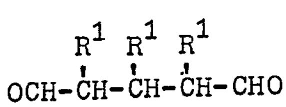

(III)

worin $R^1$ die vorgenannte Bedeutung besitzt und $R^2$ einen aliphatischen Rest bezeichnet, mit Ammoniumsalzen in Gegenwart von aliphatischen Carbonsäuren, dadurch gekennzeichnet, daß man die Umsetzung mit Ammoniumnitrat in Abwesenheit von Kupfersalzen und in Gegenwart von katalytischen Mengen Salpetersäure durchführt.

**Claim**

A process for the preparation of a pyridine of the formula I

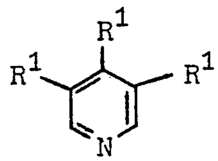
(I)

where the individual radicals $R^1$ are identical or different and each is hydrogen or an aliphatic radical, by reacting

a) a 2-alkoxy-2,3-dihydro-4H-pyran of the formula II

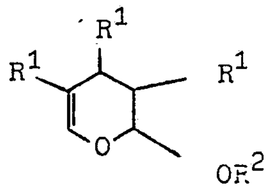
(II) or

b) a glutaraldehyde of the formula III

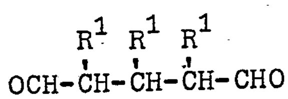
(III)

where $R^1$ has the above meanings and $R^2$ is an aliphatic radical, with an ammonium salt in the presence of an aliphatic carboxylic acid, wherein the reaction is carried out with ammonium nitrate in the absence of a copper salt and in the presence of a catalytic amount of nitric acid.

**Revendication**

Procédé pour la préparation de pyridines de formule I

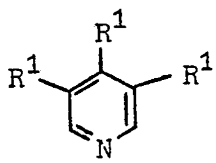
(I)

dans laquelle les différents radicaux $R^1$ peuvent être semblables ou différents et représentent chacun un atome d'hydrogène ou un radical aliphatique, par réaction de

a) 2-alcoxy-2,3-dihydro-4H-pyrannes de formule II

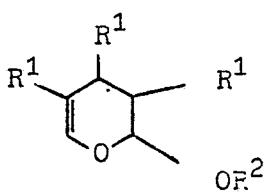
(II)

b) ou d'aldéhydes glutariques de formule III

$$OCH-\overset{R^1}{\overset{|}{C}}H-\overset{R^1}{\overset{|}{C}}H-\overset{R^1}{\overset{|}{C}}H-CHO \qquad (III)$$

dans lesquelles $R^1$ a la signification donnée ci-dessus et $R^2$ est un radical aliphatique, avec des sels ammoniques en présence d'acides carboxyliques aliphatiques, caractérisé en ce qu'on effectue la réaction avec le nitrate d'ammonium en l'absence de sels de cuivre et en présence de quantités catalytiques d'acide azotique.